# EUROPEAN PATENT APPLICATION

(11) **EP 1 281 772 A1**
(43) Date of publication of application: **05.02.2003**
(21) Application number: 01118417.3
(22) Date of filing: 31.07.2001
(51) Int. Cl.: C12Q 1/66, C12Q 1/02, A61K 49/00, C12N 15/03

(54) **Light-emitting microorganisms and cells for tumour diagnosis/therapy**

(71) Applicant: Szalay, Aladar A., Highland, CA 92346-6213 (US)
(72) Inventor: Szalay, Aladar A., Highland, CA 92346-6213 (US)
(74) Representative: Schüssler, Andrea, Dr.

(57) **Abstract**

Described are diagnostic and pharmaceutical compositions comprising a microorganism or cell containing a DNA sequence encoding a luminescent and/or fluorescent protein. Moreover, the use of said microorganism or cell for tumor-targeting or tumor-imaging is described. For therapeutic uses, said microorganism or cell additionally contain an expressible DNA sequence encoding a protein suitable for tumor therapy, e.g. a cytotoxic or cytostatic protein.

## Description

The present invention relates to diagnostic and pharmaceutical compositions comprising a microorganism or cell containing a DNA sequence encoding a luminescent and/or fluorescent protein. The present invention also relates to the use of said microorganism or cell for tumor-targeting or tumor-imaging. For therapeutic uses, said microorganism or cell additionally contain an expressible DNA sequence encoding a protein suitable for tumor therapy, e.g. a cytotoxic or cytostatic protein.

Presence of bacteria in tumors was reported approximately fifty years ago. Several publications substantiated the earlier clinical findings that unexpectedly large numbers of bacteria were discovered in excised tumors from human patients. Investigators argue that chronic infections may predispose cells to malignant growth. Chronic infections of various strains of *Chlamydia* have been associated with lung and cervical cancer as well as malignant lymphoma. Another well described association between the presence of a specific bacterial species and cancer development is *Helicobacter pylori* in patients with gastric ulcers. Elevated levels *of H. pylori*-associated antibodies have been found in patients with duodenal ulcer and gastric adenocarcinoma. These observations demonstrate a concomitant presence of bacteria at tumor sites; however, it was yet not clear whether the microorganisms were the cause of tumor formation or whether the tumorous tissues were more susceptible to bacterial colonization. Intravenously injected strict anaerobic bacteria, *Clostridium pasteurianum*, into mice replicated selectively in the tumor suggesting a hypoxic microenvironment in the necrotic center. Intravenous injection of attenuated *Salmonella typhimurium* mutants resulted in elevated bacterial titers in the tumor tissues in comparison to the other organs of mice upon histologic and bacteriologic analyses.

Similarly, the presence of virus particles was reported in excised human breast tumors as early as 1965. More recently, based on polymerase chain reaction (PCR) data, the human papillomavirus has been claimed to be associated with anogenital tumors and esophageal cancers, breast cancers, and most commonly, cervical cancers. In addition, the presence of hepatitis C virus in human hepatocellular carcinoma, Epstein-Barr virus in squamous cell carcinoma in Kirnura's disease, mouse mammary tumor virus-like paiticles (MMTV) in human breast cancer, SV40 virus in macaque astrocytoma, and herpesvirus in turtle fibropapilloma has also been reported. Surprisingly, the concentration of virus particles in the tumors shows variations among patients. The presence of human papillomavirus in squamous cell carcinomas of the esophagus ranges from 0 to 72% (10- 15). In contrast to tumor tissues, no virus particles have been found in tumor-free areas of the esophageal epithelium of the same patient suggesting that the virus particles are located only in the tumor tissues.

However, so far it could not undoubtedly been shown whether the above discussed microorganisms are responsible for the development of disorders like tumors (except for papillomaviruses) or whether, e.g., tumors can attract and/or protect viruses or bacteria. Accordingly, there was no basis for the use of such microorganisms for the diagnosis or therapy of tumors. Conventional tumor diagnostic methods, such as MRI (Magnetic Resonance Imaging) and therapeutic methods, e.g. surgery, are invasive and not very sensitive.

Therefore, it is the object of the present invention to provide a means for the efficient and reliable diagnosis as well as the therapy of tumors which overcomes the disadvantages of the diagnostic and therapeutic approaches presently used.

According to the present invention this is achieved by the subject matters defined in the claims. When *Vaccinia* virus (LIVP strain) carrying the light emitting fusion gene construct rVV-ruc-gfp was injected intravenously into nude mice, the virus particles were found to be cleared from all internal organs within 4 days, as determined by extinction of light emission. In contrast, when the fate of the injected *Vaccinia* virus was similarly followed in nude mice bearing tumors grown from subcutaneously implanted C6 rat glioma cells, virus particles were found to be retained over time in the tumor tissues, resulting in lasting light emission. The presence and amplification of the virus-encoded fusion proteins in the same tumor were monitored in live animals by observing GFP fluorescence under a stereomicroscope and by collecting luciferase-catalyzed light emission under a low-light video-imaging camera. Tumor-specific light emission was detected 4 days after viral injection in nude mice carrying subcutaneous C6 glioma implants ranging in size from 25 to 2500 mm³. The signal became more intense after the 4th postinjection day and lasted for 30 to 45 days, indicating continued viral replication. Tumor accumulation of rVV-ruc-gfp virus particles was also seen in nude mice carrying subcutaneous tumors developed from implanted PC-3 human prostate cells, and in mice with orthotopically implanted MCF-7 human breast tumors. Further, intracranial C6 rat glioma cell implants in immunocompetent rats and MB-49 human bladder tumor cell implants in C57 mice were also targeted by the *Vaccinia* virus. Cross sections of a C6 glioma revealed that light emission was clustered in "patches" at the periphery of the tumor where the fast-dividing cells reside. In contrast, cross sections of breast tumors revealed that fluorescent "islands" were distributed throughout the tumors. In addition to primary breast tumors, small metastatic tumors were also detected externally in the contralateral breast region, as well as in nodules on the exposed lung surface, suggesting metastasis to the contralateral breast and lung. In summary, the light-emitting Vaccinia virus can be used to detect primary and metastatic tumors.

Similar results were obtained with light-emitting bacteria *(Salmonella, Vibrio, Listeria, E. coli)* which were injected intravenously into mice and which could be visualized in whole animals under a low light imager immediately. No light emission was detected twenty-four hours after bacterial injection in both athymic (nu/nu) mice and immunocompetent C57 mice as a result of clearing by the immune system. In the cutaneous wound of an intravenously injected animal, the bacterial light emission increases and remains detectable up to six days post-injection. In nude mice baring tumors developed from implanted C6 glioma cells, light emission was abolished from the animal entirely twenty-four hours after delivery of bacteria, similar to mice without tumors. However, forty-eight hours post-injection, unexpectedly, a strong, rapidly increasing light emission originated only from the tumor regions was observed. This observation indicates a continuous bacterial replication in the tumor tissue. The extent of light emission is dependent on the bacterial strain used. The homing-in process together with the sustained light emission was also demonstrated in nude mice carrying prostate, bladder, and breast tumors. In addition to primary tumors, metastatic tumors could also be visualized as exemplified in the breast tumor model. Tumor-specific light emission was also observed in immunocompetent C57 mice with bladder tumors as well as in Lewis rats with brain glioma implants. Once in the tumor, the light-emitting bacteria were not observed to be released into the circulation and to re-colonize subsequently implanted tumors in the same animal. Further, mammalian cells expressing the Ruc-GFP fusion protein, upon injection into the bloodstream, were also found to home into and propagate in glioma tumors.

These findings open the way for (a) designing multifunctional viral vectors useful for the detection of tumors based on light emission and/or for suppression of tumor development and/or angiogenesis signaled by light extinction and (b) the development of bacterium- and mammalian cell-based tumor targeting systems in combination with therapeutic gene constructs for the treatment of cancer.

Accordingly, the present invention relates to a diagnostic or pharmaceutical composition comprising a microorganism or cell containing a DNA sequence encoding a luminescent and/or fluorescent protein.

Any microorganism or cell is useful for the diagnostic method of the present invention, provided that they replicate in the organism, are not pathogenic for the organism e.g. attenuated and, are recognized by the immune system of the organism, etc.

As used herein, the term "DNA sequence encoding a luminescent and/or fluorescent protein" also comprises a DNA sequence encoding a luminescent and fluorescent protein as fusion protein.

Preferably, for transfecting the cells the DNA sequences encoding a luminescent and/or fluorescent protein are present in a vector or an expression vector. A person skilled in the art is familiar with examples thereof. The DNA sequences can also be contained in a recombinant virus containing appropriate expression cassettes. Suitable viruses that may be used in the diagnostic or pharmaceutical composition of the present invention include baculovirus, vaccinia, sindbis virus, Sendai virus, adenovirus, an AAV virus or a parvovirus, such as MVM or H-1. The vector may also be a retrovirus, such as MoMULV, MoMuLV, HaMuSV, MuMTV, RSV or GaLV. For expression in mammals, a suitable promoter is e.g. human cytomegalovirus "immediate early promoter" (pCMV). Furthermore, tissue and/or organ specific promoters are useful. Preferably, the DNA sequences encoding a luminescent and/or fluorescent protein are operatively linked with a promoter allowing high expression. Such promoters, e.g. inducible promoters are well-known to the person skilled in the art.

For generating the above described DNA sequences and for constructing expression vectors or viruses which contain said DNA sequences, it is possible to use general methods known in the art. These methods include e.g. in vitro recombination techniques, synthetic methods and in vivo recombination methods as described in Sambrook et al., Molecular Cloning, A Laboratory Manual, 2^{nd} edition (1989) Cold Spring Harbor Laboratory Press, Cold Spring Harbor, NY, for example. Methods of transfecting cells, of phenotypically selecting transfectants and of expressing the DNA sequences by using the above described vectors are known in the art.

The person skilled in the art knows DNA sequences encoding luminescent or fluorescent proteins that can be used in the diagnostic or pharmaceutical of the present invention. During the past decade, the identification and isolation of structural genes encoding light-emitting proteins from bacterial luciferase from *Vibrio harveyi* (Belas et al., Science 218 (1982), 791-793) and from *Vibrio fischerii* (Foran and Brown, Nucleic acids Res. 16 (1988), 177), firefly luciferase (de Wet et al., Mol. Cell. Biol. 7 (1987), 725-737), aequorin from *Aequorea victoria* (Prasher et al., Biochem. 26 (1987), 1326-1332), *Renilla* luciferase from *Renilla reniformis* (Lorenz et al., PNAS USA 88 (1991), 4438-4442) and green fluorescent protein from *Aequorea victoria* (Prasher et al., Gene 111 (1987), 229-233) have been described that allow the tracing of bacteria or viruses based on light emission. Transformation and expression of these genes in bacteria allows detection of bacterial colonies with the aid of the low light imaging camera or individual bacteria under the fluorescent microscope (Engebrecht et al., Science 227 (1985), 1345-1347; Legocki et al., PNAS 83 (1986), 9080-9084; Chalfie et al., Science 263 (1994), 802-805).

Luciferase genes have been expressed in a variety of organisms. Promoter activation based on light emission, using *lux AB* fused to the nitrogenase promoter, was demonstrated in Rhizobia residing within the cytoplasm of cells of infected root nodules by low light imaging (Legocki et al., PNAS 83 (1986), 9080-9084; O'Kane et al., J. Plant Mol. Biol. 10 (1988), 387-399). Fusion of the *lux A* and *lux B* genes resulted in a fully functional luciferase protein (Escher et al., PNAS 86 (1989), 6528-6532). This fusion gene *(Fab2)* was introduced into *Bacillus subtilis* and *Bacillus megatherium* under the xylose promoter and then fed into insect larvae and was injected into the hemolymph of worms. Imaging of light emission was conducted using a low light video camera. The movement and localization of pathogenic bacteria in transgenic arabidopsis plants, which carry the pathogen-activated PAL promoter-bacterial luciferase fusion gene construct, was demonstrated by localizing *Pseudomonas* or *Ervinia spp*. infection under the low light imager as well as in tomato plant and stacks of potatoes (Giacomin and Szalay, Plant Sci. 116 (1996), 59-72).

All of the luciferases expressed in bacteria require exogenously added substrates such as decanal or coelenterazine for light emission. In contrast, while visualization of GFP fluorescence does not require a substrate, an excitation light source is needed. More recently, the gene cluster encoding the bacterial luciferase and the proteins for providing decanal within the cell, which includes *luxCDABE* was isolated from *Xenorhabdus luminescens* (Meighen and Szittner, J. Bacteriol. 174 (1992), 5371-5381) and *Photobacterlum leiognathi* (Lee et al., Eur. J. Biochem. 201 (1991), 161-167) and transferred into bacteria resulting in continuous light emission independent of exogenously added substrate (Fernandez-Pinas and Wolk, Gene 150 (1994), 169-174). Bacteria containing the complete *lux* operon sequence, when injected intraperitoneally, intramuscularly, or intravenously, allowed the visualization and localization of bacteria in live mice indicating that the luciferase light emission can penetrate the tissues and can be detected externally (Contag et al., Mol. Microbiol. 18 (1995), 593-603).

Preferably, the microorganism is a bacterium, e.g. attenuated Particularly preferred is attenuated *Salmonella thyphimurium*, attenuated *Vibrio cholerae or* attenuated *Listeria monocytogenes* or *E.coli*. Alternatively, viruses such as *Vaccinia* virus, AAV, a retrovirus etc. are also useful for the diagnostic and therapeutic compositions of the present invention. Preferably, the virus is Vaccinia virus

Preferably, the cell of the diagnostic or therapeutic composition of the present invention is a mammalian cell such as stem cells which can be autologous or heterologous concerning the organism.

In a further preferred embodiment of the diagnostic or therapeutic composition of the present invention the luminescent or fluorescent protein is a luciferase, green fluorescent protein (GFP) or red fluorescent protein (RFP).

In a particularly preferred embodiment, the microorganism or cell of the diagnostic or pharmaceutical composition of the present invention additionally contains a gene encoding a substrate for the luciferase. In an even more preferred embodiment, the microorganism or cell of the diagnostic or pharmaceutical composition of the present invention contains a *ruc-gfp* expression cassette which contains the *Renilla* luciferase (ruc) and Aequorea gfp cDNA sequences under the control of a strong synthetic early/late (PE/L) promoter of *Vaccinia* or the *luxCDABE cassette.*

A preferred use of the microorganisms and cells described above is the preparation of a diagnostic composition for tumor-imaging. The diagnostic composition of the present invention can be used e.g. during surgery, to identify tumors and metastasis. Furthermore, the diagnostic composition of the present invention is useful for monitoring a therapeutic tumor treatment. Suitable devices for analysing the localization or distribution of luminescent and/or fluorescent proteins in an organism, organ or tissue are well known to the person skilled in the art and, furthermore described in the literature cited above as well as the Examples, below. Additionally, the microorganisms and cells can be modified in such a way that they bind metalls and consequently are useful in MRI technology to make this more specific.

The present invention also relates to a pharmaceutical composition containing a microorganism or cell as described above, wherein said microorganism or cell furthermore contains one or more expressible DNA sequence(s) encoding (a) protein(s) suitable for tumor therapy and/or elimination of metastatic tumors, such as a cytotoxic protein, a cytostatic protein, a protein inhibiting angiogenesis, or a protein stimulating apoptosis. Furthermore, the protein can be an enzyme converting an inactive substance administered to the organism into an active substance, i.e. toxine, which is killing the tumor or metastasis. The above proteins are well-known to the person skilled in the art. Furthermore, the microorganism or cell can contain a BAC (Bacterial Artificial Chromosome) encoding several or all proteins of a specific pathway, e.g. anti-angionesis, apoptosis or woundhealing-pathway.

For administration, the microorganisms or cells of the present invention are preferably combined with suitable pharmaceutical carriers. Examples of suitable pharmaceutical carriers are well known in the art and include phosphate buffered saline solutions, water, emulsions, such as oil/water emuslions, various types of wetting agents, sterile solutions etc.. Such carriers can be formulated by conventional methods and can be administered to the subject at a suitable dose. Administration of the microorganisms or cells may be effected by different ways, e.g. by intravenous, intraperetoneal, subcutaneous, intramuscular, topical or intradermal administration. The preferred route of administration is intravenous injection The route of administration, of course, depends on the nature of the tumor and the kind of microorganisms or cells contained in the pharmaceutical composition. The dosage regimen will be determined by the attending physian and other clinical factors. As is well known in the medical arts, dosages for any one patient depends on many factors, including the patient's s size, body surface area, age, sex, the particular compound to be administered, time and route of administration, the kind, size and localization of the tumor, general health and other drugs being adminstered concurrently.

Preferred tumors that can be treated with the microorganisms or cells of the present invention are bladder tumors, breast tumors, prostate tumors, glioma tumors, adenocarcinomas, ovarial carcinomas, and pancreatic carcinomas; liver tumors, skin tumors.

### Brief description of the drawings

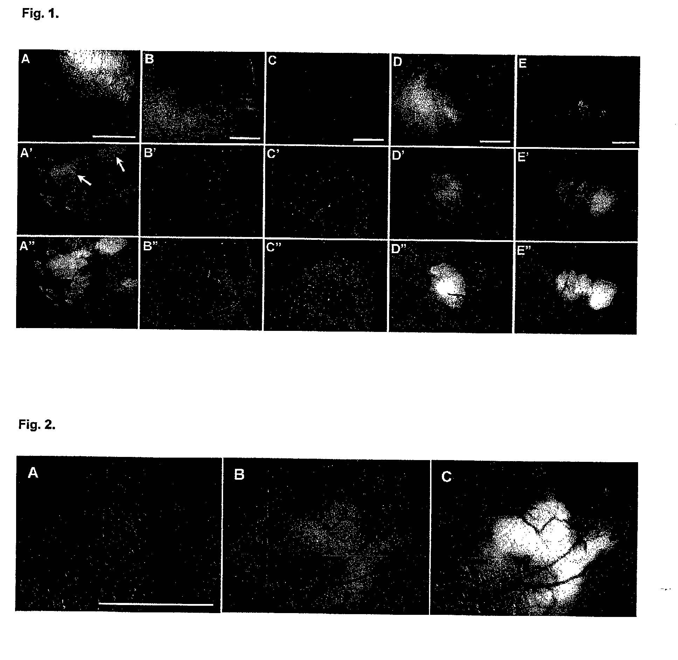

Figure 1: External imaging of GFP expression in subcutaneous C6 glioma tumors in nude mice
   C6 glioma cells (5 x 10⁵) were implanted subcutaneously into the right lateral thigh. At designated days after tumor cell implantation, the animals were infected intravenously with 1 x 10⁸ pfu of rVV-ruc-gfp virus particles. GFP expression was monitored under a fluorescence stereomicroscope. Bright field (top), fluorescence (middle), and bright field, fluorescence overlay (bottom) images of subcutaneous glioma tumor are shown. GFP signal can be observed in tumors as small as 22 mm³ in size (B-B''), or as old as 18 days (about 2500 mm³ in size) (A-A''). In older tumors, GFP expression was seen in "patch"-like patterns (indicated by arrows in A'). Marker gene expression in the tumor of the same animal can be monitored continuously 4 (C-C''), 7 (D-D''), and 14 (E-E'') days after intravenous viral injection. (Bars = 5 mm.)
Figure 2: Visualization of tumor angiogenesis
   C6 glioma cells (5 x 10⁵) were implanted subcutaneously into the right lateral thigh of nude mice. Ten days after tumor cell implantation, the animals were infected intravenously with 1 x 10⁸ pfu of rVV-ruc-gfp. GFP expression was monitored 7 days post-viral injection. Vascularization at the surface of the subcutaneous C6 glioma tumor is shown against the bright green fluorescent background in the tumor following *Vaccinia*-mediated gene expressions. Bright field (A), fluorescence (B), and bright field, fluorescence overlay (C) images of subcutaneous glioma tumor are illustrated. (Bars = 5 mm.)

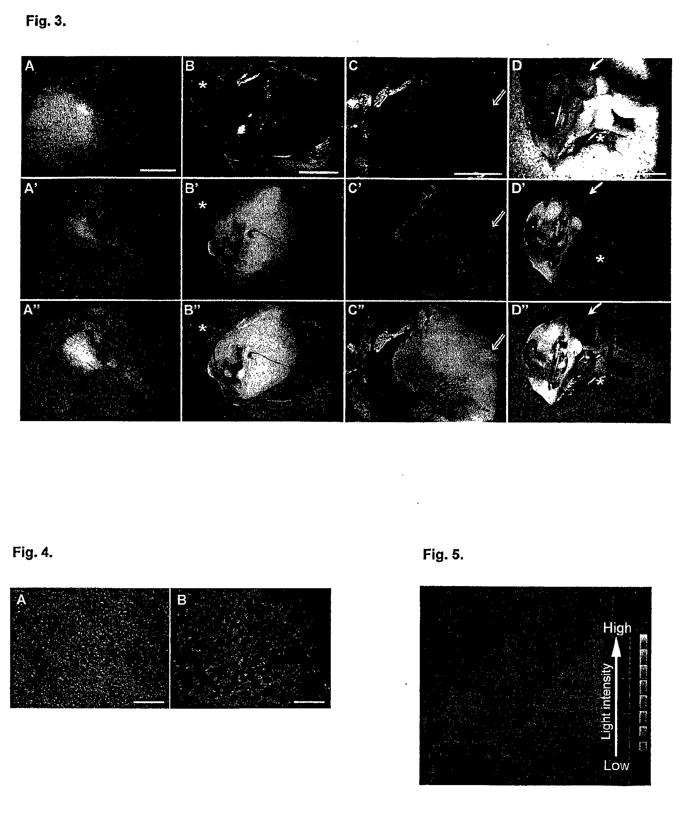

Figure 3: Expression of GFP in subcutaneous glioma tumor of the same animal
   Five days after the subcutaneous implantation of 5 x 10⁵ C6 glioma cells into the right lateral thigh, 10⁸ of rVV-ruc-gfp virus particles were injected intravenously. Five days after viral injection, the animal was anesthetized and sacrificed for analysis of GFP expression under fluorescence microscope. The tumor was visualized externally (A-K), with the overlying skin reflected (B-B''), in cross section (C-C''), and in the amputated leg (D-D''). Bright field (A), fluorescence (B), and bright field, fluorescence overlay (C) images of subcutaneous glioma tumor are illustrated. The strongest GFP expressions are seen as patches located along the outer surface of the tumor on the right (double arrows in C-C''). Sharp difference of GFP expression in tumor tissue and in the normal muscle tissue (arrows in D-D'') is clearly visible. Asterisks mark the reflected skin (B-B'' and D-D''). (Bars = 5 mm.)
Figure 4: Bright field (A) and fluorescence (B) images of tumor cells expressing GFP
   Frozen sections (30 µm thick) of the glioma tumor tissues were prepared from a nude mouse that has been intravenously injected with 1 x 10⁸ of rVV-rucgfp virus particles. (Bars = 50 µm.)
Figure 5: Low light image of the anesthetized nude mouse to indicate the location of Renilla luciferase-triggered light emission in the presence of intravenously injected substrate coelenterazine (5 µg ethanol solution)
Figure 6: Monitoring tumor-specific viral infection based on GFP gene expression in a variety of tumor models
   including subcutaneous PC-3 human prostate tumor (A-A'') and MCF-7 human breast tumor (B-B'') in nude mice, intracranial C6 rat glioma tumor (C-C'', arrows indicate the location of the tumor) in Lewis rats, and MB-49 human bladder tumor (D-D'') in C57 mice. Animals were monitored 7 days after intravenous injections of 1 x 10⁸ of rVV-ruc-gfp virus particles. Bright field (top), fluorescence (middle), and bright field, fluorescence overlay (bottom) images of the tumor are illustrated. (Bars = 5 mm.)

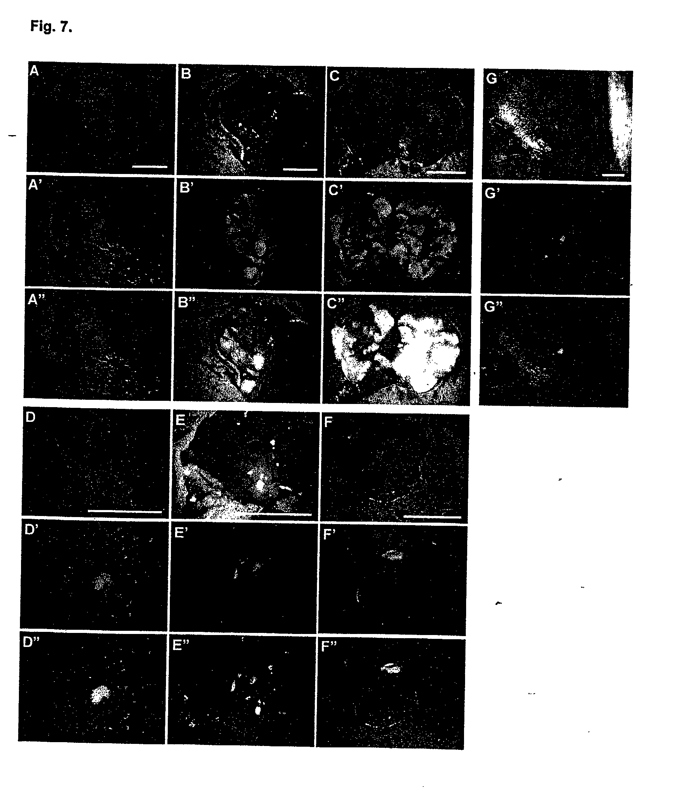

Figure 7: Monitoring Vaccinia-mediated GFP expression in a breast tumor model
   Nude mouse carrying breast tumor was injected intravenously with 1 x 10⁸ of rVV-ruc-gfp virus particles. Both the primary tumor (A-A'', B-B'', and C-C'') and the metastasized tumor (D-D'', E-E'', and F-F'') were visualized externally (A-A'' and D-D''), with overlying skin removed (B-B'' and E-E''), and when they were split open (C-C'' and F-F'') in a set of bright field, fluorescence (') and bright field, fluorescence overlay ('') images. GFP expression in lung metastases in the same animal was also visualized (G-G''). (Bars = 5 mm (A-A'' to F-F''), and Bars = 1 mm (G-G'').

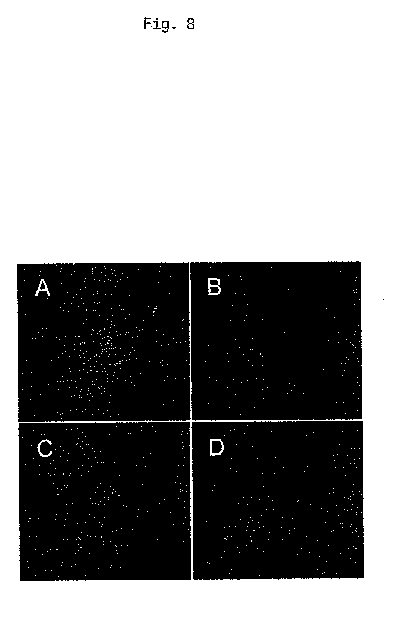

Figure 8: Visualization of the clearance of light emitting bacteria from nude mice based on the detection of light emission under the low light imager
   Nude mice were intravenously injected with 10⁷ cells of attenuated *S. typhimurium* (A, B) and *V. cholera* (C, D). Both strains were transformed with pLITE201 carrying the *lux* operon. Photon collection was done 20 min (A, C) and 2 days (B, D) after bacterial injections.

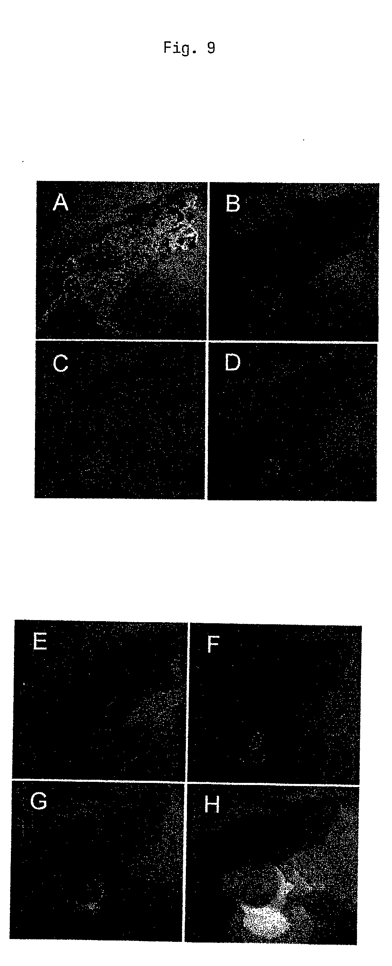

Figure 9: Homing of glioma tumors by attenuated bacteria
   Nude mice with a C6 glioma tumor in the right hind leg were intravenously injected with 10⁷ attenuated *S. typhimurium* (A-D) and with *V. cholera* (E-H) both transformed with pLITE201 plasmid DNA encoding the *lux* operon. Photon collection was carried out for one minute under the low light imager. Mice injected with *S. typhimurium* exhibited luminescence immediately through the whole animal (A) . In contrast, luminescence in the mice injected with *V. cholera* was visible in the liver area (E). Two days after bacterial injection, both groups of mice demonstrated luminescence only in the tumor region (B, F). The light emission in the tumors infected with *S. typhimurium* slowly diminished four (C) and six (D) days after bacterial injection. Tumors infected with *V. cholera* showed enormously increased light emission four (G) and six (H) days after injection suggesting continued replication of the bacteria in the tumor tissues.

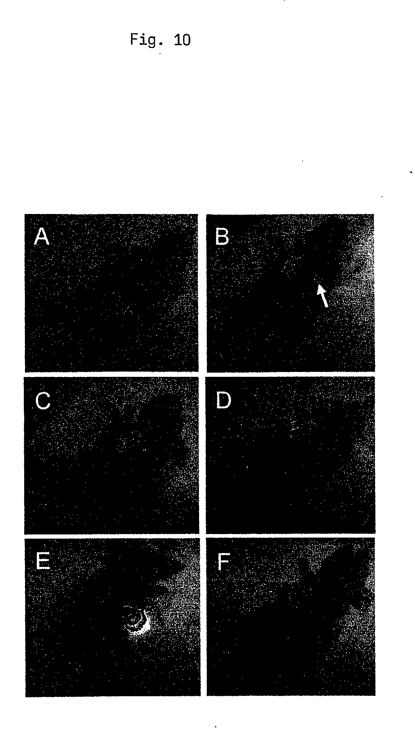

Figure 10: Homing in of bacteria onto breast tumors
   Nude mice with breast tumors in the right breast pad were intravenously injected with 10⁷ attenuated *V. cholera* (A-D) and with 10⁷ *E. coli* (E-F) transformed with pLITE201 plasmid DNA encoding the *lux* operon. Photon collection was carried out for one minute under the low light imager. Twenty minutes after bacterial delivery, luminescent *V. cholera* were observed in the liver (A). Forty-eight hours after injection, light emission was noted in the primary breast tumor in the right breast area and a metastatic tumor (arrow) in the left breast area, and in the incision wound (B). At five days, the light emission was visible only in the tumor regions, and non at the wound (C). Eight days after bacterial injection, the luminescent activity was abolished from the smaller tumor region but remained strong in the primary breast tumor (D). Homing in of *E. coli* onto breast tumors in nude mice was also observed two days after intravenous bacterial injection (E: side view, F: ventral view).

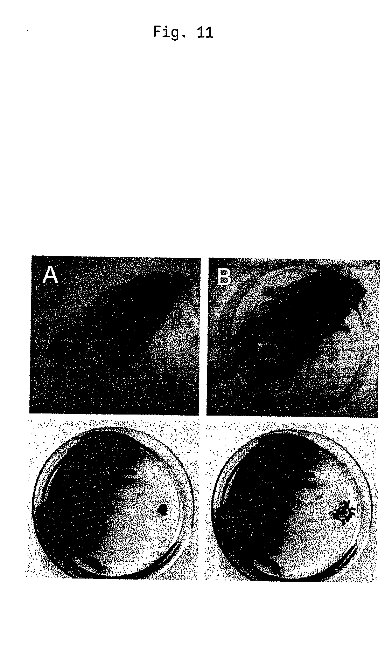

Figure 11: Homing in of bacteria onto bladder tumors in C57 mice
   C57 mice were intravenously injected with 10⁷ attenuated *V. cholera* transformed with pLITE201 encoding the *lux* operon. Nine days after bacterial delivery, luminescence was noted in the bladder region of the whole animal (A). The animal was sacrificed and an abdominal incision was made to expose the bladder. The light emission was limited to the bladder region (B). With the removal of the bladder (C) from the mouse, the entire source of light emission was removed (D) as demonstrated by the overlay of the low light photon emission image over the photographic image of the excised bladder.

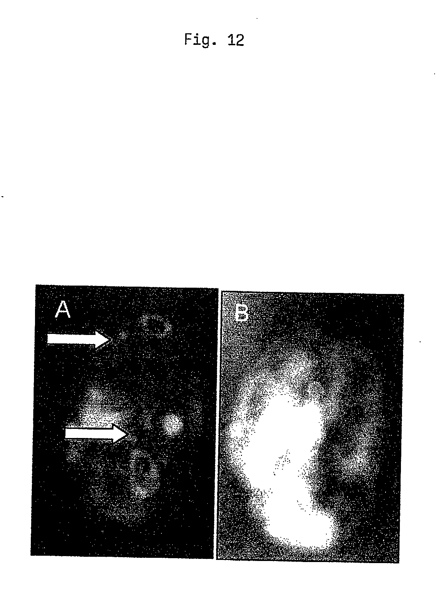

Figure 12: Homing in of bacteria onto brain glioma tumors in Lewis rats
   Lewis rats were intravenously injected with 10⁸ cells of attenuated *V. cholera* transformed with pLITE201 encoding the *lux* operon. Twenty-four hours after bacterial injection, faint luminescence was noted in the head region of the whole animal during visualization under the low light imager. The animals were sacrificed and their brain removed. Photon collection was carried out for one minute from rats with (A) and without (B) brain tumors. Strong luminescence was confirmed in regions of the brain of the rats with the brain tumor (marked with arrows in A). Luminescence was completely absent in the control brain tissues (B).

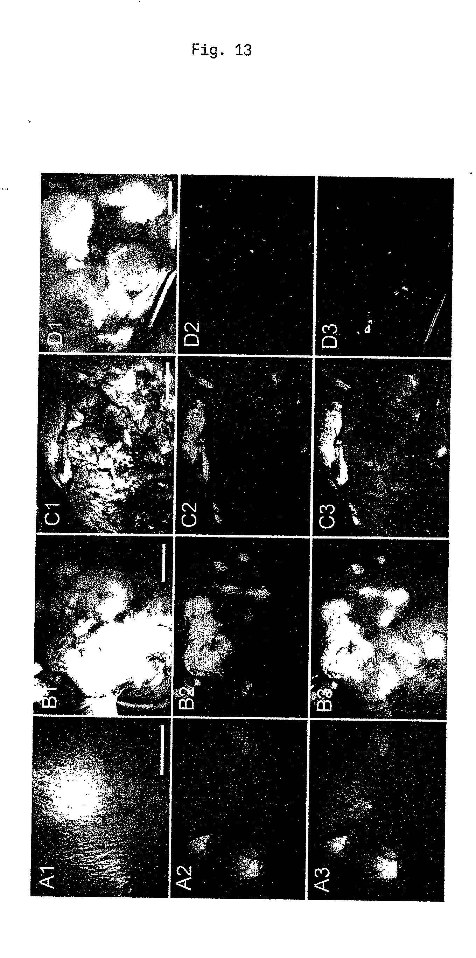

Figure 13: Transformed human fibrosarcoma cells home in on subcutaneous glioma tumors in nude mice
   Nude mice with human breast tumors were injected intravenously with 5 x 10⁵ human fibrosarcoma cells, which were permanently transformed with retrovirus derived from pLEIN. Seven days post-injection, the animals were anesthetized and monitored under a fluorescent stereomicroscope. Fluorescent cells were noted only in the tumor region of the whole mice through the skin (Al-3). Upon exposure of the tumor tissues by reflection of the overlying skin (B1 -3), and in cross sections of the tumors (C1 -3), fluorescent patches were visible in distinct regions. Close examination of the organs of the mice showed the presence of small clusters of fluorescent cells in the lungs of the animals, demonstrating the affinity of the fibrosarcoma cells for the lungs in addition to the tumorous tissues (D1-3). (Bars = 5 mm (A1-C3), = 1 mm (D1-D3)).

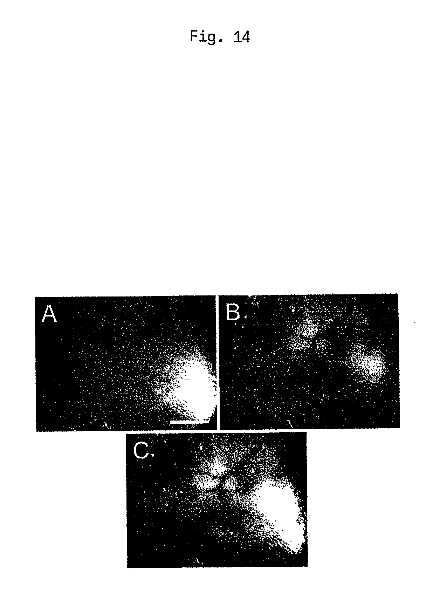

Figure 14: Homing of attenuated *Listeria monocytogenes* into subcutaneous prostate tumors
   Nude mice with subcutaneous human PC3 prostate tumor in the right hind leg were intravenously injected with 10⁷ attenuated *L. monocytogenes* transformed with pSOD-gfp plasmid DNA carrying the gfp cDNA, GFP fluorescence was observed under a fluorescence stereo microscope. Twenty-seven hours after bacterial injection, GFP signal was detected only in the tumor region. The tumor is shown in a set of visible light (a), fluorescent (b), and visible and fluorescent light overlay (C) images. (Bars = 5 mm.)

The present invention is explained by the examples.

### Example 1: Materials and Methods

**(A) Bacterium strains.** The bacterial strains used were attenuated *Salmonella typhimurium* (SL7207 hisG46, DEL407[aroA544::Tn10]), attenuated *Vibrio cholerae* (Bengal 2 Serotyp 0139, M010 DattRS1), and attenuated *Listeria monocytogenes* (D2 mpl, actA, plcB).The bacterial strains were kindly provided by Prof. W. Göbel (University of Würzburg, Germany).
**(B) Plasmid constructs.** The plasmid pLITE201 containing the *luxCDABE* cassette was obtained from (Voisey and Marincs, Biotech 24,1998, 56-58). The plasmid pXylA-dual with the operon sequence of *gfp*-cDNA, *lux* AB, lux *CD,* and *lux* E under the control of the Xylose promoter was kindly provided by Dr. Phil Hill (University of Nottingham, UK).
**(C) Transformation of bacteria**
   The bacteria were transformed by electroporation.
**(D) Tumor Cell lines.** The rat C6 nitrosourea-induced glioma cell line (ATCC, Rockille, MID) was cultured in RPMI-1640 medium (Cellgro, Mediatech, Inc., Herndon, VA) supplemented with 10% (v/v) FBS and 1 x penicillin/streptomycin. The human PC3 prostate carcinoma cell line (ATCC, Rockville, MD) and the Human MB-49 bladder tumor cells and rat 9L glioma cells were maintained in DMEM medium (Cellgro, Mediatech, Inc., Herndon, VA) supplemented with L-glutamine and 10% (v/v) FBS. HT1080 fibrosarcoma cells (ATCC, Manassas, VA) were cultured in F12 minimal essential media (Cellgro, Mediatech, Inc., Herndon, VA) supplemented with 10% FBS and 1 x penicillin/streptomycin. The MCF-7 human mammary carcinoma cell line (ATCC, Rockville, MD), permanently transformed with a plasmid carrying pro-IGF-II cDNA (obtained from Dr. Daisy De Leon, Loma Linda Iuniversity, Loma Linda, CA) was cultured in DMEM/F12 medium supplemented with 5% FBS and 560 µg/ml of G418 (Life Technologies, Grand Island, NY).
**(E) Production and propagation of retrovirus to generate a light-emitting stably transformed cell line.** PT67 packing cells (Clontech, Palo Alto, CA) were cultured in DMEM medium supplemented with 10% (v/v) FBS. At 70% confluence, PT67 cells were transformed with pLEIN (Clontech, Palo Alto, CA) using calcium phosphate precipitation method (Profection Mammalian Transfection Systems, Promega, Madison, WI) for 12 hours. Fresh medium was replenished at this time. Retroviral supernatant collected from PT67 cells 48 hours post transformation were filtered through a 0.45 µm filter and was added to target HT1080 cells along with polybrene to a final concentration of 4 µg/ml. The medium was replaced after 24 hours and the cells were treated with G418 selection at 400 µg/ml and stepwise increased to 1200 µg/ml.
**(F) Recipient animals and tumor models.** Five- to six-week-old male BALB/c athymic nu/nu mice (25-30 g in body weight) and Lewis rats (250-300 g in body weight) were purchased from Harlan (Frederick, MD). C57BL/6J *Min*/*+* mice were obtained from Jackson Laboratories (Bar Harbor, ME), *Min* (multiple intestinal neoplasia) is an autosomal dominant trait involving a nonsense mutation in codon 850 of the murine *Apc* gene, which renders these animals susceptible to spontaneous intestinal adenoma formation. Female BALB/c athymic nu/nu mice baring MCF-7 human breast tumor implants were generated and kindly provided by Dr. Daisy DeLeon and Dr. Tian (Loma Linda University, Loma Linda, CA). C57 mice with orthotopically implanted human MB-49 tumor cells in the bladder were generated and kindly provided by Dr. Istvan Fodor (Loma Linda University, Loma Linda, CA). All animal experiments were carried out in accordance with protocol approved by the Loma Linda University animal research committee. The animals containing recombinant DNA materials and attenuated pathogens were kept in Loma Linda University animal care facility under biosafety level two.
**(G) Propagation of recombinant Vaccinia virus.** *Vaccinia* virus Lister strain (LIVP) was used as a wild type virus. Recombinant *Vaccinia* virus rVV-ruc-gfp was constructed by inserting, via homologous recombination, the ruc-gfp-cassette into the *Vaccinia* virus genome (Wang et al., Proc. Biolumin. Chemilumin. 9, 1996, 419-422). The virus was amplified in CV-1 cells by addition of virus particles at a multiplicity of infection (MOI) of 0. 1 pfu/cell to CV-1 cell monolayers followed by incubation at 37°C for 1 h with brief agitation every 10 min. At this time, the supernatant fluid with virus particles was removed, and the cell monolayers were washed once with serum free medium. Complete growth medium was then added and the cells were incubated at 37°C. rVV-ruc-gfp virions propagated in CV-1 cells were purified through a sucrose gradient. A plaque assay was used 72 h after infection to determine the titer of recombinant virus by staining the cells with 50% crystal violet solution in ethanol.
**(H) Generation of mice carrying tumor implants.** To obtain tumors in nude mice, C6 glioma cells were grown, harvested and the cell number was determined by the Trypan Blue exclusion method. Disinfectant was applied to the skin surface, then approximately 5 x 10⁵ cells were suspended in 100 µl of phosphate buffered saline (PBS) and injected subcutaneously into the right lateral thigh of each mouse. Tumor growth was monitored by recording the size of the tumor with a digital caliper. Tumor volume (mm³) was estimated by the formula (L x H x W)/2, where L is the length, W is the width, and H is the height of the tumor in mm.
   Intracerebral glioma tumors were generated by injecting C6 glioma cells into the head of rats. Prior to injection, rats were anesthetized with sodium pentobarbital (Nembutal® Sodium solution, Abbot Laboratories, North Chicago, IL; 60 mg/kg body weight). A midline scalp incision (0.5 -1 cm) was made, the skin was retracted, and a 1 mm burr hole was made in the skull at a location 2 mm to the left and 2.5 mm posterior to the brigma. Tumor cells were pipetted into an insulin syringe, which was fitted with a 29-gauge needle and mounted in a stereotactic holder. The needle was inserted vertically through the burr hole to a depth of 3 mm. After injection into the brain of 5 x 10⁵ C6 cells in a 10 µl volume, the needle was kept in place for 15 sec and then withdrawn. The skin incision was closed with surgical clips. Mice bearing subcutaneous prostate tumors were generated over a period of one month following subcutaneous implantation of 3 x 10⁶ PC3 human prostate cells.
   MB-49 human bladder tumor cells were implanted in the C57 mouse bladder to produce animals with bladder tumors. To generate animals with breast cancer (Tian and DeLeon, submitted for publication), female nude mice were first implanted with 0.72 mg/90 day-release 17β-estradiol pellets (Innovative Research, Rockville, MD) in the skin to facilitate breast tumor development and metastasis. One day after estrogen pellet implantation, 1 x 10⁶ MCF-7 human breast carcinoma cells transformed with pro-IGF-II (Dull et al., Nature 310 (1984), 777-781) were implanted in the mammary fat pad. For orthotopic transplants, tumors developed from implanted cells were resected and minced into 1-mm³ cubes for tissue transplantation into the mammary fat pad.
**(I) Assay of *Renilla* luciferase in live animals.** Mice were anesthetized with Nembutal (60 mg/kg body weight) before every *Renilla* luciferase assay. *Renilla* luciferase activities were determined after intravenous injection of a mixture of 5 µl of coelenterazine (0.5 µg/µl diluted ethanol solution) and 95 µl of luciferase assay buffer (0. 5 M NaCl; 1 mM EDTA; and 0. 1 M potassium phosphate, pH 7.4). Whole live animals were then imaged in a dark box using a Hamamatsu low light video camera, and the images were recorded using Image Pro Plus 3.1 software (Media Cybernetics, Silver Spring, MD). The pseudocolored photon emission image was superimposed onto the gray scale image of the animal in order to precisely locate the site of light emission.
**(J) Fluorescence microscopy of live animals.** Mice were anesthesized with Nembutal (60 mg/kg body weight) before tumor visualization. External imaging of GFP expression in live animals was performed using a Leica MZ8 stereo fluorescence microscope equipped with a mercury lamp power supply and a GFP filter (excitation at 470 nm). Images were captured using a SONY DKC-5000 3CCD digital photo camera.
**(K) Detection of luminescence and fluorescence.** Immediately before imaging, mice and rats were anesthetized with Nembutal® (60 mg/kg body weight). The animals were placed inside the dark box for photon counting and recording superimposed images (ARGUS 100, Hamamatsu, Hamamatsu, Japan). Photon collection was for one minute from ventral and dorsal views of the animals. A light image was then recorded and the low light image was then superimposed over the light image to record the location of luminescent activity.
   Imaging of GFP expression in tumors of live animals was performed using a Leica MZ8 stereo fluorescence microscope equipped with a mercury lamp power supply and a GFP filter (excitation at 470 run). Images were captured using a SONY DKC-5000 3CCD digital photo camera.
**(L) Histology of tumor tissues.** Under anesthesia, the animals were euthanized with an overdose of Nembutal®. The tissues of interest were removed, embedded in Tissue-Tek OCT compound (Miles Scientific, Naperville, IL) and immediately frozen in liquid nitrogen without fixation. Frozen sections were cut at -20°C using a Reichert-Jung Cryocut 1800 cryostat. GFP fluorescence of the tissues was monitored under a Leica fluorescence microscope and the images were recorded using Photoshop software.

### Example 2: Results obtained by intravenous injection of recombinant Vaccinia virus rVV-ruc-gfp into mice

### (A) Monitoring of virus-mediated marker gene expression in immunodeficient mice

Vaccinia virus (1 x 10⁸ pfu) carrying the Renilla luciferase - GFP fusion expression cassette (rVV-ruc-gfp) was introduced intravenously into nude mice with no tumors. The animals were observed once every 3 days over a two-week time period under the low-light imager to monitor luciferase catalyzed light emission immediately after intravenous injection of coelenterazine, and under a fluorescence microscope to visualize GFP expression. Neither apparent luminescence nor green fluorescence was detected in the animals when imaged externally, except at certain locations that had small skin lesions. Such luminescence and fluorescence signals disappeared after a few days once the lesions had healed. Animals were sacrificed one week and two weeks after viral infection, and their organs were removed and examined for the presence of luminescence and GFP fluorescence signals. One week after viral injection, no luminescence or green fluorescence could be detected in brain, liver, lung, spleen, kidney or testis. These results indicated that the rVV-ruc-gfp virus did not show organ specificity after injection and that the virus seemed to be cleared from the animal by the immune system soon after systemic delivery via the bloodstream.

### (B)Visualization of Vaccinia virus-mediated marker gene expression in glioma tumors of live nude mice

The distribution of injected *Vaccinia* virus in nude mice bearing subcutaneously implanted C6 glioma tumors was examined. Nude mice with tumors approximately 500 mm³ in size were injected intravenously with 1 x 10⁸ pfu of the rVVruc-gfp virus. Seven days after virus injection, the animals were monitored for GFP expression under a fluorescence microscope to determine the presence of viral infection and multiplication in the tumors, which had grown to approximately 2500 mm³ in size. Surprisingly, green fluorescence was detected only in the tumor regions in live animals. Seven days after viral injection, the GFP fluorescence was very intensely localized in a patch-like pattern restricted to the tumor region (Fig. 1A-A''). These patches, often seen at the end of blood vessel branches, may have indicated local viral infection of tumor cells that surround the leaky terminals of capillary vessels. During real-time observation of the same tumors, the GFP signal from the center of these patches started to disappear, and new green fluorescent centers appeared in the form of rings at the periphery of the fading patches. The new sites of intense GFP fluorescence may have resulted from progression of the viral infection to nearby cells within the tumor during tumor growth and expansion. After careful examination of the mice, with the exception of the tumor region, no detectable green fluorescence was seen elsewhere on the body surface or in the dissected organs. This experiment clearly showed that a mature solid tumor could be easily localized by the labeled *Vaccinia* virus, based on light-emission, and it also demonstrated the affinity of virus particles for the tumor tissue.

To determine whether tumor size and vascularization are decisive factors for viral retention in tumors, nude mice were intravenously injected with 1 x 10⁸ rVV-ruc-gfp *Vaccinia* virus particles one day after subcutaneous C6 cell implantation. Surprisingly, 4 days after viral injection GFP expression was seen in 5-day-old C6 tumors that had a volume of about 25 mm³ (Fig. 1B-B''). Examination of labeled *Vaccinia* virus tumor targeting by visualization of GFP expression in implanted tumors younger than 5 days was not feasible in live mice, since sufficient levels of marker gene expression required approximately 4 days to allow detection under a fluorescence microscope.

The finding that injection of the rVV-ruc-gfp *Vaccinia* virus into the bloodstream of the host resulted in GFP expression and accumulation in tumors suitable for non-invasive tumor detection allowed us to follow the entry and replication process of this virus in the same animal in real time (Fig. 1 C-C'', D-D'' and E-E''). A continuously increasing level of GFP fluorescence was observed in the same animal throughout 20 days following viral injection, which was the time scheduled before sacrificing the animals. Such an increase in detectable fluorescence was indicative of a very strong viral replication in the tumor tissue, the latter appearing to function as a protective immunoprivileged environment for viral replication. Interestingly, the location of blood vessels and the neovascularization within the periphery of the enlarging tumor were readily visible and confirmed by external visualization against a bright green fluorescent background (Fig. 1A-A'', D-D'',E-E'' and Fig. 2).

To determine the location of viral infection within the tumors, the animals were sacrificed and the skin over the tumor was carefully reflected to expose the tumor. In the exposed tumor, GFP fluorescence was found to be concentrated exclusively in the tumor tissue (Fig. 3B-B'' and D-D''). The non-tumorous thigh muscles did not show any fluorescence of viral infection, as indicated by arrows in Fig. 3D-D''. The skin overlying the tumor was also non-fluorescent (indicated by asterisks in Fig. 3B-B'' and D-D''). Cross sections of the tumor, however, revealed that strong green fluorescent regions were mostly found as patches in the periphery of the tumor (double arrows in Fig. 3 C-C'') where the actively dividing tumor cells are presumably located.

To further examine the pattern of viral infection in C6 glioma tumors based on GFP expression, the tumor tissues were sectioned for microscopic analysis under the fluorescence microscope. Comparative analysis of various tissue sections revealed that GFP fluorescence was present in large clusters of cells within the tumor (Fig. 4), but no fluorescence was visible in normal tissues such as the heart, lung, liver, spleen, and kidney.

In addition to GFP, the recombinant rVV-ruc-gfp virus carried a second marker gene, which encoded the Renilla luciferase in the form of a fusion protein with GFP. Therefore we were able to directly superimpose the site of GFP fluorescence with light emission from *Renilla* luciferase in the tumors. Immediately after coelenterazine (substrate for *Renilla* luciferase) was delivered by intravenous injection, a very strong luciferase activity was recorded only in the tumor region under a low light video camera (Fig. 5). By lowering the sensitivity of the low light video camera to avoid saturation of light detection, we were able to identify the *Renilla* luciferase gene expression in localized patches in the periphery of the tumor. These patch-like patterns precisely correlated with the GFP signals.

### (C) Affinity of Vaccinia virus delivered to the bloodstream for different tumors implanted into animals

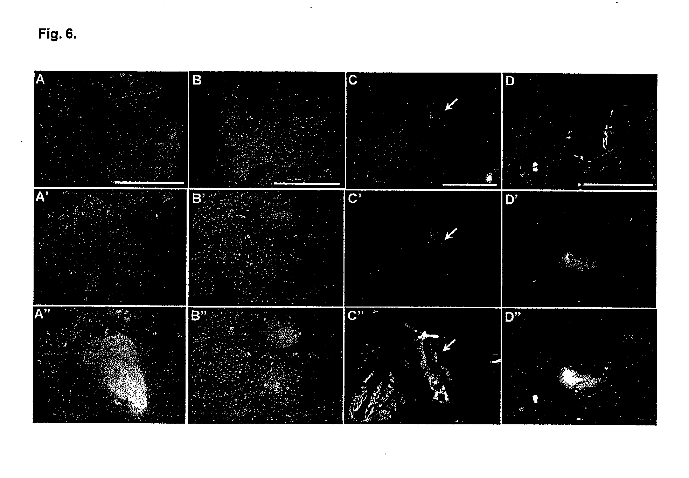

To determine whether the attraction of the *Vaccinia* virus was limited to glioma tumors or whether this attraction could be observed in other tumors, recombinant *Vaccinia* virus was recombinantly introduced into mice that carried different types of implanted tumors. One of these tumor models was a nude mouse with implanted subcutaneous PC-3 human prostate carcinoma. Although the PC3 implants from which tumors developed grew at a much slower rate than the implanted subcutaneous glioma tumors, these tumors showed the same dynamics with regards to *Vaccinia* virus infection when identical titers (1 x 10⁸) were injected intravenously (Fig. 6A-A''). Similar to our findings with glioma tumors, GFP expression was initially detected 4 days after virus injection, and the fluorescence lasted throughout the 3-week observation period.

Female nude mice with established breast tumors were also used for labeled *Vaccinia* injections. These breast tumors were allowed to grow for 6 months after the animals received implants of MCF-7 human breast carcinoma cells transformed with pro-IGF-11 cDNA. At the time of *Vaccinia* virus injection, the tumors had reached maximum growth and the tumor volume (about 400-500 mm³) did not change significantly during the experimental period. Similar to previous experiments, 6 days after intravenous delivery of 1 x 10⁸ rVV-ruc-gfp virus particles, strong GFP expression was observed in the breast tumor region (Fig. 6B-B'', Fig. 7A-A'' and B-B'') and nowhere else in the body.

Examination of cross sections of virus-infected breast tumors revealed luminescent "islands" throughout the tumors without any indication of central or peripheral preference of infection (Fig. 7C-C''). The MCF-7 tumor cells used in these breast tumor models are known to metastasize and in addition to the primary solid tumor, a smaller metastasized tumor found on the left lateral side of the body showed GFP fluorescence (Fig. 7 D-D'', E-E'', and F-F''). Excised lung tissues were also examined for detection of metastases. Metastasized tumors as small as 0.5 mm in diameter on the surface of the lung were positive for GFP fluorescence (Fig. 7G-G''). The presence of a strong *Renilla* luciferase-mediated light emission confirmed the expression of the luciferase-GFP fusion protein in these breast tumors but nowhere else in the body when the substrate coelenterazine was injected intravenously into the live animals. These experiments showed that intravenously delivered Vaccinia virus particles were selectively attracted to and replicated in primary and metastasized breast tumors in nude mice, likely as a result of the immunocompromised state of the tumor microenvironment.

To determine whether virus particles could move out of tumors and re-enter the circulation, we injected C6 glioma cells into the thigh of mice to form a second tumor in animals already carrying a breast tumor infected with labeled *Vaccinia* virus. If the virus particles were released from the tumor to re-enter the circulation in significant numbers they would be able to colonize the newly implanted glioma tumor. Monitoring of these second tumors showed that no GFP signal was visible in the new glioma tumor 7 and 14 days after implantation of the glioma cells. To demonstrate that the newly implanted glioma tumors could be targeted by labeled *Vaccinia* virus, a second dose of rVV-ruc-gfp virus (1 x 10⁸ pfu) was intravenously injected. Five days later, tumor-specific GFP expression was detected in the newly formed glioma tumor in addition to GFP expression seen in the original breast tumor. These findings suggested that the virus particles in infected tumors were either not released back into the circulation at all, or were not released in sufficient numbers to infect and replicate in a second tumor.

Two additional tumor models, including Lewis rats with intracranial C6 rat glioma tumors and C57 mice with MB-49 human bladder tumors in the bladder, were used for *Vaccinia* injections. To determine whether tumor-affinity of virus particles is a phenomenon limited to tumors in nude mice with a diminished T-lymphocyte function or whether it is a general protective property of tumors that may be demonstrated also in immunocompetent animals, Lewis rats with intracranial C6 rat glioma tumors and C57 mice with MB-49 human bladder tumors in the bladder were used. A total of 5 x 10⁵ C6 glioma cells in a 100 µl volume were stereotactically implanted in the brains of 2 of 4 immunocompetent Lewis rats, and the tumors were allowed to grow for 5 days. The other 2 rats were injected intracranially with phosphate-buffered saline to serve as controls. On day six, all 4 rats were intravenously injected with rVV-ruc-gfp virus particles via the femoral vein. Five days after virus injection, all 4 animals were sacrificed, and their brains were carefully excised for analysis by fluorescence microscopy. GFP expression was detected in the brains with implanted intracranial tumors (Fig. 6C-C'') while no GFP expression was seen in the control brains. In parallel experiments, C57 mice, with or without bladder tumors, were divided into two groups. One group was injected intravenously with rVV-ruc-gfp *Vaccinia* virus (1 x 10⁸ pfu) and the other with saline solution as control. Five days after virus injection, the animals were sacrificed and examined under the fluorescence microscope. GFP expression was observed in the bladder tumor region in C 57 mice but not in control mice (Fig. 6D-D'').

Taken together, these experiments show that *Vaccinia* virus particles were selectively accumulated and retained in a variety of tumors, probably protected by the tumor microenvironment, and that they were not able to survive in the non-tumorous tissues of immunocompromised as well as immunocompetent animals. The tumor-targeting process by intravenously injected *Vaccinia* virus carrying the light-emitting dual marker gene demonstrated the ability of the *Vaccinia* virus system to detect primary and metastatic tumors in live animals.

### Example 3: Results of intravenous injection of bacterial and mammalian light-emitting cells into mice

### (A) Visualization of light emitting bacteria present in whole animals after intravenous injection

To determine the fate of intravenously injected luminescent bacteria in the animals, 10⁷ bacteria carrying the pLITE201 plasmid in 50 µl were injected into the left femoral vein under anesthesia. Following closure of the incision with sutures, the mice were monitored under the low light imager (ARGUS 100 Camera System, Hamamatsu, Hamamatsu, Japan) in real time and photons were collected for one minute. The imaging was repeated in two-day time intervals to determine the presence of light emission from a given animal. It was found that the distribution pattern of light emission following an intravenous injection of bacteria into mice was characteristic of the bacterial strains used. Injection of the attenuated *V. cholera* into the bloodstream resulted in light emission localized in the liver immediately. Injection of *S. typhimurium*, however, was widely disseminated throughout the body of the animal suggesting a difference in the interaction with host cell system (Figure 8A-8D). Imaging the same animals 24 and 48 hours post-infection showed that all of the detectable light emission from the earlier time diminished rapidly and was eliminated completely from the injected animal. These findings suggest that light emitting bacteria injected into the bloodstream via the femoral vein are cleared. This process was confirmed by photon emission analysis of excised organs, which were found to lack light emission. Similar data were obtained in immunocompetent mice and rats suggesting that the removal of bacteria from the blood is efficient in both systems.

### (B) Bacteria home in on glioma tumors in nude mice

To determine if bacteria preferentially colonize tumorous tissues, nude mice with ten-day-old tumors (about 500 mm³) in the tight hind leg were injected intravenously via the femoral vein with 10⁷ *S. typhimurium* or 10⁷ *V. cholera* in a 50 µl volume of bacterial suspension. Following injection, the incision wounds were sutured and the animals were monitored for six days under the low light imager. At each observation time point, photons were collected for exactly one minute. In mice injected with *S. typhimurium*, luminescent bacteria were disseminated throughout the whole body of the animal similar to the findings in the non-tumorous mice (Figure 9A). Nude mice injected with *V. cholera,* demonstrated luminescent activity only in the liver region during the early observation period (Figure 9E). Regardless of the bacterial strain injected, two days after injection, luminescent activity was observed only in the tumor region (Figures 9B and 9F). Monitoring of the mice under the low light imager on days four and six post-injection showed decreased amounts of detectable luminescence in the tumors of animals injected *with S. typhimurium* (Figures 9C and 9D. This finding was in marked contrast with the findings in the tumors of mice injected with *V. cholera,* which demonstrated not only survival but also propagation of the bacteria in the tumor mass with a dramatic increase in light emission (Figures 9G and 9H).

Nude mice bearing subcutaneous human PC3 prostate tumors in the right hind leg were intravenously injected with 10⁷ attenuated *L. monocytogenes* transformed with pS0D-gfp plasmid DNA carrying the gfp cDNA. GFP fluorescence was observed under a fluorescence stereomicroscope. Twenty-seven hours after bacterial injection, GFP signal was detected only in the tumor region (Figure 10). No GFP signal was observed in the rest of the animal.

### (C) Determination of minimum size and age of glioma tumors necessary for bacterial infection.

The purpose of this experiment was to determine whether the size of the tumor has any influence on its ability to be colonized by bacteria. Tumors were induced in the right hind leg of nude mice by subcutaneous injection of glioma cells as described. On days 0, 2, 4, 6, 8, and 10 of tumor induction, attenuated *S. typhimurium* and *V. cholera* with the pLITE201 plasmid were injected intravenously through the femoral vein. Presence of luminescent bacteria in the tumor was determined by photon collection for exactly one minute under the low light imager two and four days post-infection. The tumor volume was also determined by measuring the dimensions with a digital caliper. The earliest time-point when luminescent activity was noted in the tumors was on day eight after tumor induction. Corresponding tumor volumes were approximately 200 mm³.

### (D) Bacteria home in on breast tumors of nude mice

In order to determine whether colonization of tumors is limited to glioma cells or whether this is a general phenomenon observed with all tumors, female nude mice baring tumors in the right breast pad were intravenously injected with 10⁷ *V. cholera* in a 50 µl volume of bacteria suspension. The animals were monitored within the first 10 minutes after inoculation under the low light imager for one minute and demonstrated the typical luminescent pattern in the liver region (Figure 11A). Two days later, while the liver had become clear of luminescent bacteria, the breast tumor was colonized by the labeled *V. cholera,* In addition to the main tumor, a metastatic tumor in the left breast demonstrated luminescent activity (Figure 11B). On day five, the animals had cleared the bacteria that colonized the incision wound, however, the tumors remained luminescent (Figure 11C). Figure 11D shows the continued colonization and propagation of the bacteria in the main tumor, while the metastatic, smaller tumor had become cleared. Luminescent activity continued for over 45 days in the right breast tumor. Similar experiments were conducted using *E. coli* to demonstrate that homing in of tumors by bacteria is not strain dependent (Figures 11E and 11F).

To determine whether the bacteria from the tumor enter the blood circulation in significant quantities to colonize other sites, a second tumor (C6 glioma) was induced in these animals in the right hind led. The tumor was allowed to grow for 10 days. No luminescent activity was observed in the glioma tumor demonstrating the absence of a significant bacteria that would cause colonization of this tumor. However, when the animal was rechallenged with 10⁷ attenuated *V. cholera* intravenously, the leg tumor showed strong luminescent activity.

The findings of these experiments demonstrate that larger tumors retain bacteria more effectively over time. Furthermore, the bacteria within the tumors do not escape into the blood in sufficient quantities to infect susceptible sites such as other tumors.

### (E) Bacteria home in on bladder tumors in immunocompetent mice

C57 mice were intravenously injected with 10⁷ attenuated *V. cholera* transformed with pLITE201 encoding the *lux* operon. On day nine after bacterial delivery, luminescent activity was recorded by photon collection for one minute under the low light imager. Light emission was noted in the bladder region of the whole animal (Figure 12A). The animals were sacrificed and an abdominal incision was made to expose the bladder. Luminescent activity was positively confirmed to be limited to the bladder (Figure 12B). Upon removal of the bladder from the mice, luminescent activity was no longer visible anywhere in the animals, however, the excised bladders continued to demonstrate light emission (Figure 12C). Based on the results of this experiment, bacteria can target tumors in immunocompetent as well as nude mice. Furthermore, the bacteria can also target smaller tumors.

### (F) Bacteria home in on glioma tumors in the brain of rats

Lewis rats with glioma tumors in the brain were intravenously injected with 10⁸ attenuated *V. cholera* with the pLITE201 plasmid through the left femoral vein to determine if bacteria can cross the blood-brain barrier and target tumors in immunocompetent animals. The whole animals were monitored for one minute under the low light imager the following day and low levels of luminescent activity was observed through the skull. The rats were sacrificed and the brain tissue was removed in one piece in order to further evaluate the exact location of the luminescent bacteria. Visualization of the excised brain under the imager demonstrated strong luminescent activity in specific regions of the brain (Figure 13A). Similar imaging of control rats without brains tumors, which were intravenously injected with the labeled bacteria, demonstrated absence of any luminescent activity (Figure 13B).

### (G) Transformed human fibrosarcoma cells home in on subcutaneous glioma tumors in nude mice

Nude mice with human breast tumors were injected intravenously with 5 x 10⁵ human fibrosarcoma cells, which were permanently transformed with retrovirus derived from pLEIN. Seven days post-injection, the animals were anesthetized Nembutal, and monitored under a fluorescent stereomicroscope. Fluorescent cells were noted only in the tumor region of the whole mice through the skin (Figure 14A1-3). Upon exposure of the tumor tissues by reflection of the overlying skin (Figure 14B1-3), and in cross sections of the tumors (Figure 14C1 -3), fluorescent patches were visible in distinct regions. Close examination of the organs of the mice showed the presence of small clusters of fluorescent cells in the lungs of the animals, demonstrating the affinity of the fibrosarcoma cells for the lungs in addition to the tumorous tissue.

## Claims

1. A diagnostic or pharmaceutical composition comprising a microorganism or cell containing a DNA sequence encoding a luminescent and/or fluorescent protein.

2. The diagnostic or pharmaceutical composition of claim 1, wherein the migroorganism is a bacterium or a virus.

3. The diagnostic or pharmaceutical composition of claim 2, wherein the virus is *Vaccinia* virus.

4. The diagnostic or pharmaceutical composition of claim 2, wherein the bacterium is attenuated *Salmonella thyphimurium*, attenuated *Vibrio cholerae,* attenuated *Listeria monocytogenes* or *E.coli*.

5. The diagnostic or pharmaceutical composition of claim 1, wherein the cell is a mammalian cell.

6. The diagnostic or pharmaceutical composition of any one of claims 1 to 5, wherein the luminescent or fluorescent protein is luciferase, RFP or GFP.

7. The diagnostic or pharmaceutical composition of any one of claims 1 to 6, wherein the microorganism or cell additionally contains a gene encoding a substrate for a luciferase.

8. The diagnostic or pharmaceutical composition of any one of claims 1 to 7 wherein the microorganism or cell binds metall.

9. Use of the microorganism or cell as defined in any one of claims 1 to 7 for the preparation of a diagnostic composition for tumor-imaging or monitoring a therapeutic tumor treatment.

10. Use according to claim 9, wherein tumor-imaging or monitoring is carried out in MRI.

11. A pharmaceutical composition containing a microorganism or cell as defined in any one of claims 1 to 7, wherein said microorganism or cell furthermore contains one or more expressible DNA sequences encoding (a) protein(s) suitable for tumor therapy and/or elimination of metastatic tumors.

12. The pharmaceutical composition according to claim 11, wherein the expressible DNA sequences are on a BAC.

13. Use of the microorganism or cell as defined in claim 11 or 12 for the preparation of a pharmaceutical composition for tumor therapy and/or elimination of a metastatic tumor.

14. Use according to claim 13, wherein the tumor is a bladder tumor, breast tumor, prostate tumor, glioma tumor, liver tumor, skin tumor, adenocarcinoma, ovarial carcinoma or pancreatic carcinoma.
